# EUROPÄISCHE PATENTSCHRIFT

(11) **EP 3 283 159 B1**
(45) Veröffentlichungstag und Bekanntmachung des Hinweises auf die Patenterteilung: **08.05.2019**
(21) Anmeldenummer: 16713864.3
(22) Anmeldetag: 30.03.2016
(51) Int. Cl.: A61M 39/08, A61L 29/04, B32B 27/08

(54) **SCHLAUCH FÜR EINEN MEDIZINISCHEN BEHÄLTER**
TUBE FOR A MEDICAL CONTAINER
TUBE POUR UN CONTENANT MÉDICAL

(30) Priorität: 13.04.2015 WO PCT/EP2015/057959
(43) Veröffentlichungstag der Anmeldung: 21.02.2018
(73) Patentinhaber: N.V. Nutricia, 2712 HM Zoetermeer (NL); RAUMEDIC AG, 95233 Helmbrechts (DE)
(72) Erfinder: WOLKENSTOERFER, Reinhold, 91077 Neunkirchen (DE); ZIEMBINSKI, Ralf, 95189 Koeditz-Brunnenthal (DE)
(74) Vertreter: Schicker, Silvia
(86) Internationale Anmeldenummer: PCT/EP2016/056912
(87) Internationale Veröffentlichungsnummer: WO 2016/165942

(56) Entgegenhaltungen:
- EP-A1- 2 620 168
- WO-A1-00/76564
- WO-A1-93/23093
- WO-A1-2008/127046
- WO-A1-2014/018877
- CN-U- 202 733 209
- US-A- 5 733 619
- US-A1- 2009 087 606
- US-A1- 2010 055 367
- US-A1- 2015 075 665

## Beschreibung

### Gebiet der Erfindung

Die vorliegende Erfindung bezieht sich auf einen Schlauch für einen medizinischen Behälter gemäß dem Oberbegriff des Anspruchs 1, insbesondere auf einen Schlauch mit einer Schlauchwand, die aus mindestens zwei Schichten besteht.

Ein Schlauch für einen medizinischen Behälter, im Folgenden auch medizinischer Schlauch genannt, zeichnet sich dadurch aus, dass der Schlauch flexibel, weich und transparent ist und dass er beim Führen um Rundungen herum bzw. beim Biegen nicht einknickt und an der Knickstelle das Lumen des Schlauches versperrt. Eine weitere wichtige Eigenschaft eines medizinischen Schlauches ist, dass der Schlauch zuverlässig und sicher elastisch wieder auffedert, nachdem er zusammengedrückt worden ist. Letztere Eigenschaft ist gleichzusetzen mit einer guten Elastizität bzw. einem guten Rückstellverhalten und wird auch als "einen guten Snap aufweisend" bezeichnet. Diese Eigenschaft ist insbesondere bei der Verwendung des medizinischen Schlauches im Zusammenwirken mit schlauchverformenden Pumpsystemen wichtig. Ein Anwendungsgebiet derartiger Schläuche ist insbesondere die parenterale bzw. enterale Ernährung von Patienten.

### Stand der Technik

Seit mehreren Jahrzehnten werden weitverbreitet medizinische Schläuche verwendet, die eine im Wesentlichen homogene Schlauchwand aus Polyvinylchlorid (PVC) mit darin eingelagerten Weichmachern enthalten. Vorteile dieser Schläuche aus PVC mit Weichmachern sind die kostengünstige Herstellung aufgrund der leichten Verfügbarkeit des Ausgangsmaterials, und dass diese Schläuche transparent sind und ein gutes Rückstellverhalten aufweisen. Ohne den Zusatz von Weichmachern wäre das Polyvinylchlorid als solches zu hart für die Verwendung in einem medizinischen Schlauch. Nachteile derartiger Schläuche sind, dass in den letzten Jahren die üblicherweise verwendeten Weichmacher für das PVC in den Verdacht geraten sind, gesundheitsgefährdend zu sein. Als weiterer Nachteil dieser Schläuche entsteht bei deren Entsorgung durch Verbrennen Salzsäure und je nach thermischen Bedingungen auch Dioxine. Deshalb ist diese Entsorgung als solche nicht umweltverträglich und muss unter besonders abgeschirmten Bedingungen erfolgen, was die Kosten der Entsorgung erhöht.

Im Rahmen der Weiterentwicklung zum Bereitstellen von beim Entsorgen umweltverträglichen Schläuchen, die keine gesundheitsgefährdenden, insbesondere keine krebserregenden Stoffe enthalten, wurden in EP 0 355 711 Schläuche vorgeschlagen, deren Schlauchwand aus homogenem Ethylenvinylacetat Copolymer (EVA) mit einem Vinylacetat(VA)-Anteil von 12 bis 28 % besteht. Diese sogenannten Monoschläuche aus EVA als Alternative zu Schläuchen aus PVC sind transparent, weich und flexibel und weisen eine gutes Rückstellverhalten (einen guten Snap) auf. Auch sind diese Monoschläuche aus EVA gut mit medizinischen Behältern verbindbar, wenn diese Behälter oder die daran befestigten Ansatzstücke für den Schlauch ebenfalls aus Ethylenvinylacetat hergestellt sind. Der medizinische Schlauch kann dann nämlich mit dem Behälter oder dessen Ansatzstück leicht verschweißt werden. Ein Nachteil von Monoschläuchen aus EVA ist, dass das EVA aufgrund seiner chemischen Inertheit nicht, insbesondere nicht mit handelsüblichen Lösungsmitteln, verklebt werden kann mit bei medizinischen Behältern üblicherweise verwendeten Materialien. Dazu gehören etwa die in Ansatzstücken bzw. Konnektoren verwendeten Materialien, die typischerweise Acrylnitril-Butadien-Styrol (ABS), Polycarbonat (PC), Polyvinylchlorid (PVC), Polymethylmethacrylat (PMMA), Copolyester, Copolyesterether, thermoplastisches Polyurethan (TPU) oder Kombinationen aus diesen Stoffen umfassen. Zum Bereitstellen von medizinischen Schläuchen, die gut mit den vorgenannten Materialien verklebbar sind, wurden in der DE 195 04 414 medizinische Schläuche vorgeschlagen, deren Schlauchwand eine Zusammensetzung aus mindestens zwei Schichten umfasst, wobei eine äußere Schicht Ethylenvinylacetat (EVA) und eine innere Schicht Polyurethan (PU) enthält. Bei diesen zweischichtigen Schläuchen dient die innere Schicht aus Polyurethan als Klebeelement, das es ermöglicht, dass der vorgeschlagene Schlauch durch Aufschieben auf einen aus einem der vorgenannten typischerweise verwendeten Materialien hergestellten, dornartig ausgebildeten Konnektor eines medizinischen Behälters gut verklebt werden kann. Ein Nachteil der zweischichtigen Schläuche mit einer Polyurethan-Schicht als Klebeschicht ist, dass dieser Schlauch im Vergleich zu herkömmlichen Schläuchen aus PVC mit Weichmachern relativ teurer in der Herstellung ist. Ein weiterer Nachteil ist, dass dieser Schlauch nur zum Verbinden mit einem dornartig ausgebildeten Konnektor durch Aufschieben auf diesen Konnektor geeignet ist.

US 8,178,647 B2 beschreibt medizinische Multischicht-Schläuche, welche eine äußere Schicht mit Ecdel 9966, eine mittlere Schicht aus EVA und eine innere Schicht aus LDPE enthalten. Diese Schläuche weisen zwar gute Klebeeigenschaften auf und können an Konnektoren verbunden werden, sind jedoch zu hart und eignen sich somit schlecht in der Verwendung als medizinische Schläuche.

Ein Schlauch gemäss dem Oberbegriff von Anspruch 1 ist aus dem Dokument US 5 733 619 A bekannt.

Allgemein sind in der herkömmlichen Verbindungstechnik von Schläuchen und medizinischen Behältern zwei Arten der Verbindung bekannt. Gemäß einer ersten Art weist der Behälter einen als Dorn bzw. dornartig ausgebildeten Konnektor auf, auf den ein medizinischer Schlauch aufgeschoben werden kann. Typischerweise wird beim Aufschieben des Schlauchs auf den dornartigen Konnektor ein Lösungsmittel als Gleitmittel verwendet, und der aufzuschiebende Schlauch weist eine durch das Lösungsmittel anlösbare innenseitige Oberflächenschicht auf. Die Haftung des Schlauchs an bzw. dessen Verklebung mit dem dornartigen Konnektor kann nach dem Aufschieben durch Längsdehnen des Schlauches verbessert werden. Durch die Längsdehnung des Schlauchs schrumpft der Schlauch bezüglich seines Durchmessers, so dass ein nach innen gerichteter Druck auf den dornartig ausgebildeten Konnektor zunimmt. Gemäß einer zweiten Art einer Verbindung zwischen einem medizinischen Behälter und einem medizinischen Schlauch weist der Behälter einen als Ansatzstutzen ausgebildeten Konnektor auf und der Schlauch ist in eine distalseitige Öffnung des Stutzens einschiebbar. Bei dieser zweiten Art kommt es wesentlich darauf an, dass eine gute Verklebung bzw. Abdichtung zwischen der äußeren Oberfläche des Schlauchs und der inneren Oberfläche des Ansatzstutzens des Konnektors erzielt wird. Da bei dieser Verbindungsart keine Dehnung des Schlauchs auftritt, ist es erforderlich, dass die Außenseite des Schlauchs eine gute Löslichkeit für ein Lösungs- bzw. ein Klebemittel aufweist und dadurch eine Verbindung mit dem Material an der Innenseite des Ansaugstutzens hergestellt wird.

Der vorliegenden Erfindung liegt die Aufgabe zugrunde, einen Schlauch für einen medizinischen Behälter bereitzustellen, der eine verbesserte Verbindbarkeit, insbesondere Verklebbarkeit, aufweist und flexibel ist.

Zur Lösung der Aufgabe wird ein Schlauch für einen medizinischen Behälter vorgeschlagen, wobei der Schlauch eine Schlauchwand aufweist, die aus mindestens zwei Schichten besteht. Erfindungsgemäß enthält mindestens eine Schicht ein Styrol-haltiges thermoplastisches Polymer (S-TPE), ein Copolyester, ein Copolyester-Ether oder ein Cyclo-Olefin-Copolymer (COC).

Thermoplastische Elastomere (lineare Elastomere; TPE) sind Kunststoffe, die sich bei Raumtemperatur vergleichbar den klassischen Elastomeren verhalten, sich jedoch unter Wärmezufuhr plastisch verformen lassen und somit ein thermoplastisches Verhalten zeigen. Thermoplastische Elastomere sind Werkstoffe, bei denen elastische Polymerketten in thermoplastisches Material eingebunden sind. Obwohl keine chemische Vernetzung durch eine zeit- und temperaturaufwendige Vulkanisation, wie bei den Elastomeren, notwendig ist, haben die hergestellten Teile aufgrund ihrer besonderen Molekularstruktur doch gummielastische Eigenschaften.

Nach dem inneren Aufbau von TPEs unterscheidet man häufig Blockcopolymere und Elastomerlegierungen. Blockcopolymere besitzen innerhalb eines Moleküls Hart- und Weichsegmente, wie z.B. Styrol-Butadien-Styrol-Blockcopolymere (SBS). Elastomerlegierungen hingegen sind Blends, also physikalische Mischungen von fertigen Polymeren. Durch unterschiedliche Mischungsverhältnisse und Additive kann man ein Material mit gewünschten Eigenschaften erhalten, wie z.B. Polyolefin-Elastomer aus Polypropylen und Naturgummi. Je nach Mengenverhältnis kann die Härte in einem weiten Bereich angepasst werden.

Styrol-haltige thermoplastische Polymere (S-TPE) können grundsätzlich wie oben ausgeführt als Blockcopolymere oder Elastomerlegierungen ausgebildet sein.

Das Styrol-haltige thermoplastische Polymer (S-TPE) kann in einer bevorzugten Ausführungsform ein Styrol-Butadien-Block-Copolymer (SBC) sein. Ein Beispiel eines solchen SBC ist, wie unten beschrieben, Polystyrol-Butadien-Polystyrol (SBS). SBC sind kommerziell gut und preiswert verfügbar, beispielsweise von der Firma BASF unter dem Markennamen Styroflex®, wobei bevorzugt das Styroflex mit der Produktbezeichnung 2G 66 verwendet wird. Im Englischen ist hierfür die Bezeichnung "SBC" gebräuchlich, während im Deutschen die Bezeichnungen "SBS" bzw. "SEBS" gebräuchlich sind.

In der vorliegenden Erfindung bevorzugt sind insbesondere Styrol-Blockcopolymere, wobei die anderen Blöcke aus Polybutadien, Polyethylenbutylen, Polyisopren und Polyisopren/Butadien-Copolymer bestehen. Beispiele von solchen S-TPEs sind Polystyrol-Butadien-Polystyrol (SBS), Polystyrol-Polyethylenbutylen-Polystyrol (SEBS), Polystyrol-Polyisopren-Polystyrol (SEPS), Polystyrol-Polyisopren/Butadien-Polystyrol (SEEPS) und Polymethylmethacrylat-Polybutadien-Polystyrol (MBS) Blockcoplymere. Dabei kann jedoch auch ein Block, bspw. der Polybutadien-Block in SBS durch ein statistisches Styrol-Butadien-Copolymer ersetzt sein. Die S-TPEs der vorliegenden Erfindung sind also nicht auf reine Block-Copolymere beschränkt.

Copolyester werden gebildet, wenn Polyester modifiziert werden, welche wiederum Kombinationen von Disäuren und Diolen sind. Als Beispiel kann das gut Bekannte Polyethylenterephthalat (PET), welches aus Terephthalsäure (TPS) und Ethylenglykol (EG) hergestellt wird, durch Einbau von anderen Monomeren, wie Isophthalsäure (IPA) oder Cyclohexandimethanol (CHDM) zu einem anderen Copolyester, wie z.B. Polycyclohexylendimethylenterephthalat (TPS+CHDM/EG) werden. Copolyester haben in der Regel gute Eigenschaften bzgl. Stärke, Transparenz und anderen mechanischen Eigenschaften, wie exzellente Zähigkeit, hydrolytische Stabilität, Hitzeresistenz, und Resistenz gegenüber Chemikalien, welche üblicherweise Polymere, wie z.B. Polycarbonate beeinflussen. Ein in dieser Erfindung verwendeter Polyester ist Tritan MX710 der Fa. Eastman, welcher die oben genannten Eigenschaften besitzt.

Copolyesterether umfassen ein Polyester-Segment und ein Polyether-Segment. Copolyesterether haben gute Eigenschaften, wie Transparenz, Zähigkeit und hohe chemische Resistenz. Dabei ist das Polyester-Segment wie oben beschrieben zusammengesetzt, d.h. es kann ein Polyester wie PET oder ein entsprechendes Copolyester sein. Das Polyether-Fragment besteht aus einem Polyether (auch Polyalkylenglycol, Polyetherpolyol, Polyalkylenoxid) oder aus einem Polyether-Polyol. Beispiele für Polyether sind Polyethylenglycol (PEG) und Polypropylenglycol (PPG), die beide durch katalytische Polymerisation der entsprechenden Epoxide (Oxirane) Ethylenoxid bzw. Propylenoxid hergestellt werden. Die entsprechende PolyetherPolyole können hergestellt werden durch Umsetzung von Epoxiden mit Diolen. Neben Diolen können auch mehrwertige Alkohole, wie z. B. Glycerin, 1,1,1-Trimethylolpropan (TMP), Pentaerythrit oder Sorbit mit Epoxiden in Gegenwart starker Basen (z. B. KOH) zu Polyether-Polyolen umgesetzt werden. Das Polyether-Fragment kann auch als Blockcopolymer vorliegen, welche durch sequentielle Polymerisation mit unterschiedlichen Epoxiden hergestellt werden. Gängige Polyetherpolyole sind Lupranol (BASF SE) und Desmophen (Bayer Material Science). Auch Epoxidharze sind Polyether mit endständigen Epoxidgruppen. Die in dieser Erfindung bevorzugten Copolyesterether sind jedoch solche die aus einem einfachen Polyester und einem einfachen Polyether zusammengesetzt sind. Ein Beispiel eines solchen, in dieser Erfindung verwendeten Copolyesterethers ist das Ecdel 9967 der Fa. Eastman.

Cyclo-Olefin-Copolymere (COC) sind Copolymere mit Olefin-Einheiten wie Ethylen und Cycloolefinen-Einheiten wie z.B. Norbornen. Norbornen wird bspw. aus Cyclopentadien und Ethylen hergestellt. Die Reaktion zum Copolymer ist üblicherweise Metallocen-Katalysiert und führt zu statistischen Copolymeren. Obwohl nur aus Olefinen bestehend, sind COC im Gegensatz zu den teilkristallinen Polyolefinen wie Polyethylen und Polypropylen amorph und damit transparent. Die Eigenschaften von COC lassen sich durch Veränderung der Einbauverhältnisse von zyklischen und linearen Olefinen in einem weiten Bereich verändern. Beispielsweise unterdrückt das sperrige Norbornen, wenn es verwendet wird, die Kristallinität und führt zu steifen Polymerketten. Flexible amorphe Copolymere können jedoch erhalten werden durch einen niedrigen Norbornen-Gehalt von weniger als 20 mol-%. Flexible semi-kristalline Copolymere können erhalten werden durch einen niedrigen Norbornen-Gehalt von weniger als 15 mol-%. Im Wesentlichen kann die Wärmeformbeständigkeit in einem Bereich von 65 bis 190 °C eingestellt werden. Allen COCs gemeinsam sind eine Reihe von Eigenschaften wie gute thermoplastische Fließfähigkeit, hohe Steifigkeit, Festigkeit und Härte sowie niedrige Dichte und hohe Transparenz bei guter Säure- und Laugenbeständigkeit. Ein in dieser Erfindung verwendetes Cyclo-Olefin-Copolymer ist das TOPAS Elastomer E-140 von Topas Advanced Polymers.

Wenn S-TPE, Copolyester, Copolyester-Ether oder Cyclo-Olefin-Copolymer für die Schicht verwendet werden, verleihen sie dem Schlauch eine gute Verbindbarkeit, insbesondere eine gute Verklebbarkeit mit den Materialien, die typischerweise zur Herstellung medizinischer Behälter bzw. daran befestigter Konnektoren verwendet werden, wie etwa ABS, PC, PVC, PMMA, Copolyester, Copolyesterether, thermoplastisches Polyurethan (TPU) oder Mischungen aus diesen Stoffen.

Bei geeigneter Wahl des Materials der anderen Schicht, beispielsweise EVA, ist ein derartiger Schlauch auch nicht teurer in der Herstellung als Schläuche aus PVC mit Weichmachern. Zusätzlich kann das EVA zur Verbesserung seines Rückstellverhaltens noch mit einem TPE (z.B. SEBS Copolymer) abgemischt werden (Blend). Auch ist ein solcher Schlauch frei von PVC und Weichmachern und dadurch beim Entsorgen umweltverträglich und nicht gesundheitsgefährdend bzw. krebserregend.

Vorzugsweise enthält der Schlauch mindestens eine Schicht, die ein Ethylen-Vinylacetat-Copolymer (EVA) enthält. Insbesondere ist diese eine Schicht nicht die eine Schicht, die das Styrol-haltige thermoplastische Polymer, Copolyester, Copolyester-Ether oder Cyclo-Olefin-Copolymer enthält. Ein Schlauch mit einer S-TPE-, Copolyester-, Copolyester-Ether- oder Cyclo-Olefin-Copolymer-enthaltenden Schicht und einer EVA-enthaltenden Schicht ist nicht teurer in der Herstellung als herkömmlich verwendete Schläuche aus PVC mit Weichmachern und ist frei von PVC und Weichmachern.

Der Vinylacetat(VA)-Anteil des Ethylen-Vinylacetat-Copolymers beträgt 10 % bis 30 Gew.%, vorzugsweise 14 % bis 28 %. Durch diese Materialwahl ist der Schlauch unempfindlich gegenüber Temperaturänderungen und weist gute mechanische Eigenschaften, wie ein gutes Rückstellverhalten, auf.

In der Ausführungsform, in der mindestens eine Schicht Ethylen-Vinylacetat-Copolymer (EVA) enthält, kann diese Schicht (zusätzlich) ein transparentes Thermoplastisches Polybuten oder wahlweise ein SEBS enthalten. Das Thermoplastische Polybuten oder SEBS kann bei der Schicht als Additiv mit einem Anteil von 1 Gew.% bis 50 Gew.%, vorzugsweise 10 Gew.% bis 20 Gew.%, hinzugefügt werden.

Der Schlauch kann so ausgebildet sein, dass die äußere der mindestens zwei Schichten ein Styrol-haltiges thermoplastisches Polymer (z.B. Styroflex 2G 66 des Herstellers Fa. Styrolution), ein Copolyester (z.B. Tritan MX710 des Herstellers Fa. Eastman) ein Copolyesterether (z.B. Ecdel 9967 des Herstellers Fa. Eastman) oder ein Cyclo-Olefin-Copolymer (z.B. Topas® Elastomer von Topas Advanced Materials) enthält. Ein derartiger Schlauch ist angepasst zum Verbinden mit einem medizinischen Behälter gemäß der zweiten Verbindungsart, d.h. zum Einschieben in einen als Ansatzstutzen ausgebildeten Konnektor des medizinischen Behälters.

Alternativ dazu kann der Schlauch so ausgebildet sein, dass die innere der mindestens zwei Schichten ein Styrol-haltiges thermoplastisches Polymer, ein Copolyester ein Copolyesterether oder ein Cyclo-Olefin-Copolymer enthält. Ein solcher Schlauch ist angepasst zum Verbinden mit einem medizinischen Behälter gemäß der ersten Verbindungsart, d.h. zum Aufschieben des Schlauchs auf einen dornartig ausgebildeten Konnektor des medizinischen Behälters.

Vorzugsweise weist die das Styrol-haltige thermoplastische Polymer, Copolyester, Copolyesterether oder Cyclo-Olefin-Copolymer enthaltende Schicht eine Dicke auf, die geringer ist als die Dicke der anderen Schicht der mindestens zwei Schichten des Schlauches. Bei geeigneter Wahl des Materials der anderen Schicht, beispielsweise EVA, weist der Schlauch gute mechanische Eigenschaften, insbesondere eine gute Elastizität, auf. Ferner ist der die vergleichsweise dünnere, das Polystyrol-haltige thermoplastische Polymer, Copolyester, Copolyesterether oder Cyclo-Olefin-Copolymer enthaltende Schicht gut angepasst zum Verkleben zwischen dieser Schicht und Materialien, die typischerweise zum Herstellen von medizinischen Behältern bzw. daran vorgesehenen Konnektoren eingesetzt werden, wie etwa Acrylnitril-Butadien-Styrol (ABS), Polycarbonat (PC), Polyvinylchlorid (PVC), Polymethylmethacrylat (PMMA), Copolyester, Copolyesterether, thermoplastisches Polyurethan (TPU) oder Kombinationen aus diesen Stoffen.

Alternativ zu einem zweischichtigen Aufbau kann die Schlauchwand aus mindestens drei Schichten bestehen, wobei die äußere und die innere der mindestens drei Schichten ein Styrol-haltiges thermoplastisches Polymer, Copolyester, Copolyesterether oder Cyclo-Olefin-Copolymer enthalten. Ein so ausgebildeter Schlauch ist angepasst zum Verkleben mit sowohl einem als Ansatzstutzen ausgebildeten als auch mit einem dornartig ausgebildeten Konnektor. Ein so ausgebildeter Schlauch kann auch verwendet werden als Adapterstück zum Verbinden der Enden zweier Schläuche mit unterschiedlichen Durchmessern oder zum Verbinden eines Schlauchs mit einem dornartig ausgebildeten Konnektor, wobei der äußere Durchmesser des Konnektors kleiner ist als der Innendurchmesser des Schlauches, oder zum Verbinden eines Schlauches mit einem als Ansatzstutzen ausgebildeten Konnektor, wobei der innere Durchmesser des Konnektors größer ist als der äußere Durchmesser des Schlauchs.

In dem mindestens drei Schichten umfassenden Schlauch kann die äußere und die innere, das Styrol-haltige thermoplastische Polymer, Copolyester, Copolyesterether oder Cyclo-Olefin-Copolymer enthaltende Schicht jeweils eine Dicke aufweisen, die geringer ist als die Dicke der mittleren Schicht der mindestens drei Schichten. Die dadurch erzielten Vorteile sind die gleichen, wie bei dem Schlauch mit zweischichtigem Wandaufbau und einer im Vergleich zur Dicke der anderen Schicht dünneren, das S-TPE enthaltenden Schicht.

Die das Styrol-haltige thermoplastische Polymer, Copolyester, Copolyesterether oder Cyclo-Olefin-Copolymer enthaltende Schicht kann eine Dicke von 0,01 bis 0,3 mm aufweisen. Eine derartig geringe Dicke genügt bereits, um vermittels des in der Schicht enthaltenen S-TPE eine gute Verklebbarkeit bereitzustellen. Darüber hinaus ist der Ausgangsmittelbedarf (Rohstoffbedarf) des S-TPE gering im Vergleich zu dem des Materials der anderen, relativ dickeren Schicht des Schlauchs.

Der Schlauch kann einen Innendurchmesser von 3,0 mm bzw. mit einer Toleranz von ±0,1 mm aufweisen. Ein derartiger Schlauch ist angepasst zur Verwendung mit bezüglich ihrer Größe, insbesondere dem Außendurchmesser, handelsüblich verfügbaren und standardisierten, dornartig ausgebildeten Konnektoren.

Der Schlauch kann einen Außendurchmesser von 4,10 mm mit einer Toleranz von +0,1 mm und -0,3 mm aufweisen. Ein derartiger Schlauch ist angepasst zur Verwendung mit handelsüblich verfügbaren und standardisierten, als Ansatzstutzen ausgebildeten Konnektoren.

Der Schlauch kann eine Wanddicke von 0,55 mm mit einer Toleranz von ±0,05 mm aufweisen. Ein derartig ausgebildeter Schlauch weist ähnlich vorteilhafte mechanische Eigenschaften auf wie die weitverbreitet eingesetzten, insbesondere standardisierten Schläuche aus PVC mit Weichmachern.

Der Schlauch kann durch Coextrusion hergestellt werden. So wird der Schlauch in einem einstufigen und damit kostengünstigen Herstellungsverfahren hergestellt.

Nach einem anderen Aspekt der vorliegenden Erfindung wird ein medizinischer Behälter bereitgestellt, insbesondere ein Behälter für die enterale oder die parenterale Ernährung. Erfindungsgemäß umfasst der medizinische Behälter mindestens einen Ansatz aus einem erfindungsgemäßen Schlauch, wie er vorstehend beschrieben ist.

Der Behälter kann aus einem Material hergestellt sein, das Ethylen-Vinylacetat-Copolymer (EVA) enthält, und der Ansatz kann mit einer äußeren Schicht des Schlauchs, dessen äußere der mindestens zwei Schichten das Styrol-haltige thermoplastische Polymer, Copolyester, Copolyesterether oder Cyclo-Olefin-Copolymer enthält, verschweißt sein.

Alternativ kann der medizinische Behälter aus einem Material hergestellt sein, das Ethylen-Vinylacetat-Copolymer (EVA) enthält, wobei der Behälter einen dornartig ausgebildeten Ansatz umfasst, auf dem ein Schlauch mit einer inneren Schicht, die ein Styrol-haltige thermoplastische Polymer, Copolyester, Copolyesterether oder Cyclo-Olefin-Copolymer enthält, aufgeschoben worden ist.

Im Folgenden werden zwei Ausführungsbeispiele der Erfindung anhand der Zeichnungen genauer beschrieben. Es stellen dar:
- Fig. 1: einen Querschnitt durch eine erste Ausführungsform eines erfindungsgemäßen Schlauches;
- Fig. 2: einen Querschnitt durch eine zweite Ausführungsform eines erfindungsgemäßen Schlauches, und
- Fig. 3: einen Querschnitt durch eine dritte Ausführungsform eines erfindungsgemäßen Schlauches.

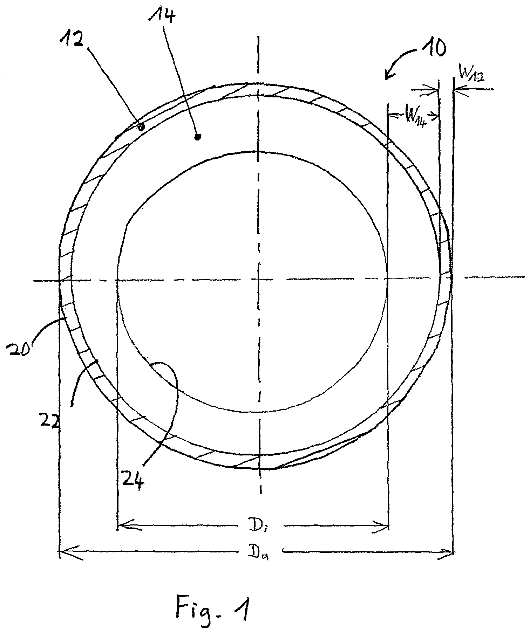

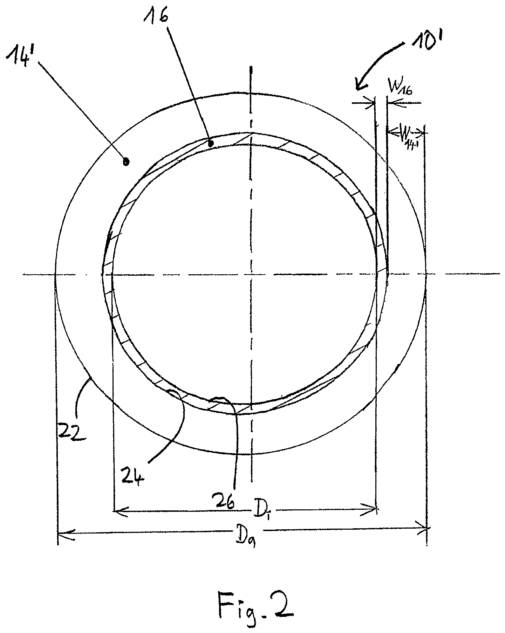

In den Figuren 1 bzw. 2 ist jeweils ein Schlauch 10 bzw. 10' mit einem zweischichtigen Aufbau der Schlauchwand dargestellt. Der in Fig. 1 gezeigte Schlauch 10 gemäß der ersten Ausführungsform der Erfindung umfasst eine äußere Schicht 12, die ein Styrol-haltiges thermoplastisches Polymer, Copolyester, Copolyesterether oder Cyclo-Olefin-Copolymer enthält, und eine innere Schicht 14, die ein von dem Material der äußeren Schicht 12 verschiedenes Material enthält. Die äußere Schicht 12 kann auch vollständig aus einem Styrol-haltigen thermoplastischen Polymer, wie beispielsweise einem Styrol-Butadien-Block-Copolymer (SBC), einem Copolyester, einem Copolyesterether oder einem Cyclo-Olefin-Copolymer hergestellt sein. Die innere Schicht 14 ist aus einem von Polyvinylchlorid (PVC) und von Polycarbonat (PC) verschiedenen Material, und vorteilhaft aus Ethylen-Vinylacetat (EVA) mit einem Vinylanteil von 10 Gew.% bis 30 Gew.%, vorzugsweise 14 Gew.% bis 28 Gew.%, hergestellt.

Der in Fig. 2 gezeigte Schlauch 10' gemäß der zweiten Ausführungsform der Erfindung umfasst eine äußere Schicht 14', die aus einem von PVC, von PC und von S-TPE verschiedenes Material hergestellt ist, und eine innere Schicht 16, die ein Styrol-haltiges thermoplastisches Polymer, Copolyester, Copolyesterether oder Cyclo-Olefin-Copolymer enthält. Für die in der inneren Schicht 16 bzw. der äußeren Schicht 14' des Schlauchs 10' in Fig. 2 enthaltenen Materialien gilt das entsprechend gleiche wie das für die in der äußeren Schicht 12 bzw. der inneren Schicht 14 des Schlauchs 10 in Fig. 1 enthaltenen Materialien Gesagte.

Die äußeren Schichten (Schicht 12 in Fig. 1 und Schicht 14' in Fig. 2) und die inneren Schichten (Schicht 14 in Fig. 1 und Schicht 16 in Fig. 2) weisen jeweils eine gleichmäßige Wandstärke auf. Eine Außenfläche einer jeweiligen inneren Schicht der Schläuche 10 in Fig. 1 und 10' in Fig. 2 steht vollflächig in Kontakt mit einer Innenfläche der jeweiligen äußeren Schicht.

Die Außenfläche und eine Innenfläche der jeweiligen inneren Schicht sowie eine Außenfläche und die Innenfläche der jeweiligen äußeren Schicht weisen jeweils einen kreisförmigen Querschnitt auf. Ferner sind sie koaxial angeordnet, also mit im Querschnitt zusammenfallenden Mittelpunkten. Die Schläuche 10 (in Fig. 1) und 10' (in Fig. 2) sind jeweils durch Coextrusion hergestellt, so dass die jeweilige äußere Schicht und die jeweilige innere Schicht fest miteinander verbunden sind. Der jeweilige Schlauch 10 bzw. 10' ist zumindest auf der Innenfläche 24 bzw. 26 der jeweiligen inneren Schicht 14 bzw. 16 des Schlauchs 10 bzw. 10' und vorzugsweise auch auf der Außenoberfläche 20 bzw. 22 der jeweiligen äußeren Schicht 12 bzw. 14' sterilisiert, beispielsweise durch eine Behandlung mit Ethylenoxid oder durch Strahlensterilisation.

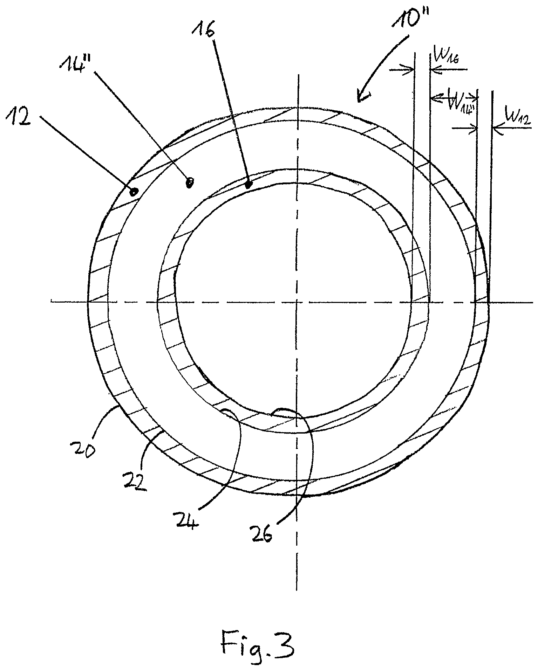

Der in Fig. 3 gezeigte Schlauch 10" gemäß der dritten Ausführungsform der Erfindung weist eine Schlauchwand mit einem dreischichtigen Aufbau auf. Die Schlauchwand umfasst eine äußere Schicht 12, die ein Styrol-haltiges thermoplastisches Polymer, Copolyester, Copolyesterether oder Cyclo-Olefin-Copolymer enthält, eine mittlere Schicht 14", die ein von PVC, PC und S-TPE verschiedenes Material enthält, und eine innere Schicht 16, die ein Styrol-haltige thermoplastische Polymer, Copolyester, Copolyesterether oder Cyclo-Olefin-Copolymer enthält. Für die Materialien der äußeren Schicht 12 und der inneren Schicht 16 gilt das gleiche wie das für die äußere Schicht 12 des Schlauchs 10 in Fig. 1 und das für die innere Schicht 16 des Schlauchs 10' in Fig. 2 Gesagte. Für das Material der mittleren Schicht 14" des Schlauchs 10" in Fig. 3 gilt das gleiche wie das für das Material der inneren Schicht 14 des in Fig. 1 gezeigten Schlauchs 10 und das Material der äußeren Schicht 14' des in Fig. 2 gezeigten Schlauchs 10' Gesagte.

In einer bevorzugten Ausführungsform wird als das Styrol-haltige thermoplastische Polymer (S-TPE) vorzugsweise das von der Firma BASF unter dem Markennamen Styroflex® verfügbare Produkt mit der Produktbezeichnung 2G 66 verwendet. In einer anderen bevorzugten Ausführungsform wird als Copolyester das von der Fa. Eastman verfügbare Produkt mit der Produktbezeichnung Tritan MX710 oder alternativ das Copolyesterether mit der Produktbezeichnung Ecdel 9967 des Herstellers Fa. Eastman verwendet. In einer weiteren bevorzugten Ausführungsform wird als Cyclo-Olefin-Copolymer das COC Topas® Elastomer von Topas Advanced Materials verwendet.

In den Ausführungsformen eines Schlauchs, in der mindestens eine Schicht ein Ethylen-Vinylacetat-Copolymer (EVA) enthält, kann bei der Herstellung dieser Schicht zusätzlich ein transparentes Thermoplastisches Polybuten (z.B. das Produkt mit der Bezeichnung Koattro KT AR05 des Herstellers Fa. Basell) oder SEBS (z.B. Kraton G1652 des Herstellers Fa. Kraton) hinzugefügt werden. Dabei kann der Gewichtsanteil des Polybutens oder SEBS 1% bis 50%, vorzugsweise 10% bis 20%, mehr bevorzugt 2% und noch mehr bevorzugt 5% betragen.

In Versuchsreihen wurde gefunden, dass sich durch die Zugabe eines Thermoplastischen Polybutens oder SEBS, als Additiv in das EVA allgemein
- die Knickanfälligkeit reduziert;
- die Eigenschaften des Schlauchs hinsichtlich seiner Biegeeigenschaften (einschließlich des "Snap") ähnlicher wie die eines Schlauchs aus PVC werden, was wünschenswert ist, weil in der Fachwelt Schläuche aus PVC als Standard angesehen und Neuentwicklungen medizinischer Schläuche mit Schläuchen aus PVC verglichen werden;
- die Elastizität verbessert, so dass der Schlauch leichter wickelbar wird (was vorteilhaft ist, weil der Schlauch zum Transport in mehreren (O-förmigen) Wicklungen auf Vorrats- bzw. Transportspulen aufgewickelt wird);
- und schließlich ein ausgeprägtes "Formgedächtnisses" (in der Fachsprache "Memory" genannt) von Schläuchen aus EVA (d.h. EVA ohne zugefügtes Additiv) verringert (was beispielsweise bei der Verwendung eines neuen (frischen) Schlauchs aufgrund des Biegeradius einer beim Transport verwendeten Spule auftritt).

Die äußere Schicht 12, die mittlere Schicht 14" und die innere Schicht 16 weisen jeweils eine gleichmäßige Wandstärke auf. Eine Außenfläche der inneren Schicht 16 steht vollflächig in Kontakt mit einer Innenfläche 24 der mittleren Schicht 14" und eine Außenfläche 22 der mittleren Schicht 14" steht vollflächig in Kontakt mit einer Innenfläche der äußeren Schicht 12. Die Außenflächen und die Innenflächen einer jeweiligen inneren, mittleren und äußeren Schicht weisen jeweils einen kreisförmigen Querschnitt auf. Sie sind koaxial angeordnet, also mit dem Querschnitt zusammenfallenden Mittelpunkten. Der Schlauch 10" ist durch Coextrusion hergestellt, so dass die äußere Schicht 12 mit der mittleren Schicht 14" und die innere Schicht 16 mit der mittleren Schicht 14" fest miteinander verbunden sind. Der Schlauch 10" ist auf der Innenfläche 26 der inneren Schicht 16 und vorzugsweise auch auf der Außenfläche 20 der äußeren Schicht 12 sterilisiert, beispielsweise durch Behandlung mit Ethylenoxid oder durch Strahlensterilisation.

Bei den in den Figuren 1 bis 3 gezeigten Schläuchen 10, 10', 10" gemäß der ersten bis dritten Ausführungsform der Erfindung beträgt der Innendurchmesser Dᵢ 3,0 mm mit einer Toleranz von ± 0,10 mm, der Außendurchmesser Dₐ 4,10 mm mit einer Toleranz von +0,10 mm und -0,30 mm und die Wandstärke W₁₂, W₁₆ der S-TPEenthaltenden Schichten 12, 16 0,01 bis 0,30 mm.

Eine in den Figuren nicht dargestellte Infusionsvorrichtung umfasst einen medizinischen Behälter und einen medizinischen Verbindungsschlauch zum Patienten. Der medizinische Behälter weist einen Beutel auf mit einem Konnektor zum Anschließen eines medizinischen Schlauchs. Der Beutel besteht aus Ethylenvinylacetat-Copolymer und ist mit einer fetthaltigen Ernährungslösung, beispielsweise zur enteralen oder parenteralen Ernährung eines Patienten, gefüllt.

In einer Ausführungsform umfasst der Behälter einen als Ansaugstutzen ausgebildeten Konnektor. Der Behälter ist aus Ethylen-Vinylacetat-Copolymer und der Konnektor aus Acrylnitril-Butadien-Styrol (ABS), Polycarbonat (PC), Polyvinylchlorid (PVC), Polymethylmethacrylat (PMMA), Copolyester, Copolyesterether, thermoplastisches Polyurethan (TPU) oder Kombinationen hergestellt. Der Konnektor ist als Ansatzstutzen ausgebildet, in den ein medizinischer Schlauch eingeführt bzw. hineingesteckt werden kann. Der Schlauch ist gemäß der in Fig. 1 gezeigten ersten oder gemäß der in Fig. 3 gezeigten dritten Ausführungsform ausgebildet und weist an seiner Außenseite eine S-TPE-enthaltende Schicht 12 auf. Die Schicht 12 des Schlauchs 10 bzw. 10" und ist mit der Innenseite des Ansatzstutzens verklebt, und zwar unter Verwendung eines handelsüblichen Lösungsmittels, wie beispielsweise Cyclohexanon (CHEX), Tetrahydrofuran (THF), Methyl-Ethyl-Keton (MEK) oder einem Gemisch aus diesen Stoffen (z.B. MIX-MEK/CHEX).

In einer anderen Ausführungsform ist der Konnektor dornartig ausgebildet und über den dornartig ausgebildeten Konnektor ist ein Schlauch 10' gemäß der in Fig. 2 gezeigten zweiten Ausführungsform oder ein Schlauch 10" gemäß der in Fig. 3 gezeigten dritten Ausführungsform des erfindungsgemäßen medizinischen Schlauchs aufgeschoben. Vor dem Aufschieben wurde ein handelsübliches Lösungsmittel auf der Innenoberfläche des Schlauchs 10, 10' oder/und auf der Außenfläche des dornartig ausgebildeten Konnektors aufgetragen und wirkt beim Aufschieben des Schlauchs auf den Konnektor als Gleitmittel. Nachdem der Schlauch auf den Konnektor aufgeschoben worden ist, wird der Schlauch zumindest in dem auf den Konnektor aufgeschobenen Bereich in seiner Längsrichtung gedehnt, so dass eine elastische, radial nach innen gerichtete Spannung bzw. ein radial nach innen gerichteter Anpressdruck auf die Außenoberfläche des dornartig ausgebildeten Konnektors entsteht. Unter Einwirkung des Lösungsmittels werden die innere Schicht 16 des Schlauchs 10' bzw. 10" und die äußere Oberfläche des dornartig ausgebildeten Konnektors angelöst und miteinander durch Klebung fest verbunden.

Zur Herstellung der Schläuche 10, 10', 10" nach der ersten, zweiten bzw. dritten Ausführungsform werden einer Coextrusionsmaschine die beiden Grundstoffe Ethylen-Vinylacetat-Copolymer oder alternativ ein Polymerblend aus EVA und Thermoplastischem Polybuten oder/und SEBS als Feststoffe, sowie die äußere und/oder innere Schicht aus Styrol-haltigem thermoplastischem Polymer (z.B. Styroflex 2G 66 des Herstellers Fa. Styrolution), Copolyester (z.B. Tritan MX710 des Herstellers Fa. Eastman), Copolyesterether (z.B. Ecdel 9967 des Herstellers Fa. Eastman) oder Cyclo-Olefin-Copolymer (z.B. Topas® Elastomer von Topas Advanced Materials) insbesondere als Granulate, getrennt voneinander zugeführt. Die Grundstoffe werden getrennt erwärmt und von je einer Extrudereinrichtung, beispielweise einer Extruderschnecke, verdichtet. Die erwärmten und zähflüssigen Massen werden getrennt einer geeignet ausgestalteten Strangpressdüse zugeführt, aus der sie bei der ersten Ausführungsform als Schlauch 10 mit einer inneren Schicht aus EVA und einer äußeren Schicht aus S-TPE, Copolyester, Copolyesterether, oder COC in der zweiten Ausführungsform als Schlauch 10' mit einer äußeren Schicht 14' aus EVA und einer inneren Schicht 16 aus S-TPE und in der dritten Ausführungsform als Schlauch 10" mit einer äußeren Schicht 12 und einer inneren Schicht 16 aus S-TPE, Copolyester, Copolyesterether, oder COC und einer mittleren Schicht 14" aus EVA austreten.

### Experimenteller Teil

Die in der Anmeldung beschriebenen Materialien für die Schläuche zum Anschluss an einen medizinischen Behälter wurden anhand von Klebeversuchen und ihrer Biegesteifigkeit hin getestet und miteinander verglichen.

### Bestimmung der Biegesteifigkeit

In dieser Versuchsreihe wurde die Durchbiegung bzw. Elastizität der Schläuche bei gegebener Last bestimmt. Hierfür wurde ein FRANK-PTI Biegesteifigkeitsprüfgerät TS verwendet.

In den nachstehenden Versuchen (siehe Tabelle 1) wurden Schläuche mit den nachfolgend angegebenen Materialien und Material-Kombinationen verwendet.

In Versuchsreihe 1 wurde PVC als getestet. Das verwendete PVC war Weich-PVC mit einer Härte von Shore A 80, Weichmacher war DINCH der Fa. BASF SE.

In Versuchsreihe 2 wurde LDPE/S-TPE als Zwei-Schicht-Schlauchmaterial verwendet. Das verwendete LDPE war das Purell PE 1840 des Herstelles Basell, das S-TPE war Styroflex 2G 66 des Herstellers Fa. Styrolution.

In Versuchsreihe 3 wurde EVA/Copolyesterether als Zwei-Schicht-Schlauchmaterial verwendet. Das verwendete EVA war Evathane 28.05 des Herstellers ARKEMA, das Copolyesterether war Ecdel 9967 der Fa. Eastman.

In Versuchsreihe 4 wurde EVA/PET als Zwei-Schicht-Schlauchmaterial verwendet.

In Versuchsreihe 5 wurde SoftPP als Schlauchmaterial verwendet. Das verwendete Soft PP war eine Abmischung aus random-Polypropylen-Copolymer und einem hydrierten Styrol/Isopren Blockcopolymer.

In Versuchsreihe 6 wurde EVA alleine als Schlauchmaterial verwendet.

Als Konnektoren in den Versuchsreihen wurden die gängigen in der Medizintechnik verwendeten Materialien Hart-PVC (Nakan RMA705N T01, Referenz), Polycarbonat (Makrolon Rx 1805) und Copolyester (Tritan MX 731) getestet.

Je Versuchsreihe wurden 10 Schläuche von jeweils ca. 10 cm Länge geprüft. Die Prüfung wird nach 72 h Ablagerung unter klimatisierten Bedingungen analog ShoreA-Prüfung (ISO 868) durchgeführt. Die Proben wurde in die vorgesehen Einspannvorrichtung eingespannt, wobei der Schlauch ca. 1 cm hinten aus der Spannvorrichtung herausstand und der größte Teil des Schlauches vorne überstand.

Die Prüfbedingungen bzw. die Parametereinstellung waren wie folgt:
23°C ± 1 °C Raumtemperatur
Messgenauigkeit ± 1%
Prüfgeschwindigkeit 6 °/s
Geschwindigkeit bis Vorkraft: 6°/s
Winkel: 30° (Angabe des Endwinkels)
Haltezeit: 2s
Vorkraft: 0,005 N
Prüfabstand: 30 mm

Die Messergebnisse wurden als gemessene maximale Kraft [N] abgelesen und wiederholt bis 10 Messergebnisse vorlagen, woraus der Mittelwert gebildet wurde.

### Bestimmung der Abzugskraft mittels Zugversuch

In dieser Versuchsreihe wurde die Abzugskraft in [N] mittels Zugversuch bestimmt.

Die verwendeten Schlauchmaterialien waren dieselben wie für die Bestimmung der Biegesteifigkeit.

Die verschiedenen Schläuche wurden jeweils mit den beschriebenen Lösungsmitteln (Tetrahydrofuran (THF) oder Mischung von Methyl-Ethyl-Keton (MEK) und Cyclohexanon (CH oder CHEX)) an die diversen Formteile geklebt und vor Durchführung der Zugversuche über 5 Tage bei Raumtemperatur gelagert bis zum vollständigen Ablüften der Lösungsmittel.

Die Probekörper waren dabei die verschiedenen Formteile, welche mit einem Schlauch verklebt wurden. Als Messgerät wurde eine Zugprüfmaschine der Fa. Zwick verwendet.

Die Prüfgeschwindigkeit betrug 200 mm/min, die Einspannlänge war Probekörperspezifisch.

### Ergebnisse

Die EN 1615/1618 spricht von einer ausreichenden Zugfestigkeit, wenn diese 15 N beträgt. Dies wird vorliegend jedoch als zu niedrig erachtet. Vorliegend wurde daher versucht, eine Zugfestigkeit von mindestens 2 X dieser Norm, also von mindestens 30 N zu erreichen.

Ausreichende Klebeeigenschaften hat ein Material z.B. dann, wenn eine Abzugskraft von mindestens ca. 35 N benötigt wird. Eine darunter liegende Abzugskraft von bsp. 20 N deutet auf eine unzureichende Stabilität der Schlauch-Konnektor-Verbindung hin.

Eine gute Elastizität hat ein Material dann, wenn es eine Biegesteifigkeit von unter ca. 0,7 mN aufweist. Materialien mit einer höheren Biegesteifigkeit sind in der Regel zu starr und nicht für die Verwendung als Schlauch für medizinische Behälter geeignet.

Es wurde gefunden, dass das üblicherweise verwendete PVC zwar gute Klebeeigenschaften aufweist, jedoch viel zu hart ist und darüber hinaus, wie Eingangs beschrieben andere Nachteile aufweist, wie z.B. darin enthaltene Weichmacher und mangelnde Umweltverträglichkeit (siehe Tabellen 1 und 2, Versuchsreihe 1).

Wird hingegen ein Schlauch aus transparentem EVA (Monoschlauch) verwendet, ist der Halt am Konnektor unzureichend, wie sich in den Klebeversuchen herausgestellt hat (siehe Tabelle 1, Versuchsreihe 7).

Auch die Verwendung von Soft-PP hat sich als unzureichend bezüglich der Klebeeigenschaften herausgestellt (siehe Tabelle 1, Versuchsreihe 6).

Zusätzlich wurden verschiedene Materialkombinationen miteinander verglichen. Die Kombination von LDPE und Styrol-haltigem thermoplastischen Polymer (Styroflex 2G 66) führt zwar zu guten Klebeeigenschaften (siehe Tabelle 1, Versuchsreihe 2), jedoch zu ungenügender Flexibilität (siehe Tabelle 2, Versuchsreihe 2).

Die Kombinationen von
- EVA mit einem Copolyesterether (Tabellen 1 und 2, Versuchsreihe 3),
   von
- EVA mit einem Styrol-haltigen thermoplastischen Polymer (Tabellen 1 und 2, Versuchsreihe 4),
   und von
- EVA mit einem Copolyester (Tabellen 1 und 2, Versuchsreihe 5),
haben alle gute elastische Eigenschaften und weisen gleichzeitig eine gute Verklebbarkeit mit den Materialien von Konnektoren auf.

Anstelle von EVA kann auch thermoplastisches Polybuten und/oder SEBS verwendet werden.

Anhand der Versuche wurde somit gezeigt, dass die Schläuche gemäß der Erfindung mit mindestens zwei Schichten, wobei eine Schicht aus S-TPE, Copolyester oder Copolyesterether besteht, den gewünschten Eigenschaften von guter Verklebbarkeit und hoher Elastizität gerecht werden. Auch die Cyclo-Olefin-Copolymere haben in hier nicht gezeigten Experimenten die gewünschten Eigenschaften gezeigt.

**Tabelle 1: Klebeversuche mit verschiedenen einschichtigen und zweischichtigen Schläuchen**

| **Probe** | | | | | **Abzugskraft (N)** | **Abzugsart** |
|---|---|---|---|---|---|---|
| **Bezeichnung Schlauch** | **Abmessung** | **Material Schlauch** | **Konnektor** | **Lösungsmittel** | | |
| Versuchsreihe 1 | 3 x 4,1 | PVC | Hart-PVC | THF | 86,1 | Schlauch reißt im Konnektor |
| | 3 x 4,1 | PVC | Hart-PVC | 50T MEK / 50T CH | 80,1 | Schlauch reißt am Konnektor |
| | 3 x 4,1 | PVC | Polycarbonat | THF | 51,4 | Schlauch zieht ab |
| | 3 x 4,1 | PVC | Polycarbonat | 50T MEK / 50T CH | 51,8 | Schlauch zieht ab |
| | 3 x 4,1 | PVC | Copolyester | THF | 81,2 | Schlauch reißt am Konnektor |
| | 3 x 4,1 | PVC | Copolyester | 50T MEK / 50T CH | 64,0 | Schlauch reißt im Konnektor |
| | | | | | | |
| Versuchsreihe 2 | 3/0,65 | LDPE/S-TPE | Polycarbonat | THF | 81,7 | Schlauch zieht ab |
| | 3/0,65 | LDPE/S-TPE | Polycarbonat | 50T MEK / 50T CH | 72,6 | Schlauch zieht ab |
| | 3/0,65 | LDPE/S-TPE | Copolyester | THF | 72,5 | Schlauch zieht ab |
| | 3/0,65 | LDPE/S-TPE | Copolyester | 50T MEK / 50T CH | 80,6 | Schlauch zieht ab |
| | | | | | | |
| Versuchsreihe 3 | 0,65 x 4,15 | EVA/Copolyesterether | Hart-PVC | THF | 46,6 | Schlauch zieht ab |
| | 0,65 x 4,15 | EVA/Copolyesterether | Hart-PVC | 50T MEK /50T CH | 54,3 | Schlauch reißt am Konnektor |
| | 0,65 x 4,15 | EVA/Copolyesterether | Polycarbonat | THF | 45,0 | Schlauch reißt am Konnektor |
| | 0,65 x 4,15 | EVA/Copolyesterether | Polycarbonat | 50T MEK / 50T CH | 40,5 | Schlauch zieht ab |
| | 0,65 x 4,15 | EVA/Copolyesterether | Copolyester | THF | 35,1 | Schlauch zieht ab |
| | 0,65 x 4,15 | EVA/Copolyesterether | Copolyester | 50T MEK / 50T CH | 27,9 | Schlauch zieht ab |
| Versuchsreihe 4 | 3/0,65 | EVA/S-TPE | Polycarbonat | THF | 36,6 | Schlauch zieht ab |
| | 3/0,65 | EVA/S-TPE | Polycarbonat | 50T MEK /50T CH | 40,5 | Schlauch zieht ab |
| | 3/0,65 | EVA/S-TPE | Copolyester | THF | 37,8 | Schlauch zieht ab |
| | 3/0,65 | EVA/S-TPE | Copolyester | 50T MEK / 50T CH | 59,6 | Schlauch zieht ab |
| | | | | | | |
| Versuchsreihe 5 | 0,65/4,15 | EVA/Copolyester | Polycarbonat | THF | 27,5 | Schlauch reißt am Konnektor |
| | 0,65/4,15 | EVA/Copolyester | Copolyester | THF | 33,2 | Schlauch reißt am Konnektor |
| | 0,65/4,15 | EVA/Copolyester | Copolyester | 50T MEK / 50T CH | 28,2 | Schlauch reißt am Konnektor |
| | | | | | | |
| Versuchsreihe 6 | 3 x 4,16 | Soft-PP | Polycarbonat | THF | 21,5 | Schlauch zieht ab |
| | 3 x 4,16 | Soft-PP | Copolyester | THF | 20,4 | Schlauch reißt am Konnektor |
| | 3 x 4,16 | Soft-PP | Copolyester | 50T MEK / 50T CH | 24,4 | Schlauch zieht ab |
| | | | | | | |
| Versuchsreihe 7 | 2,98 x 4,18 | EVA | Polycarbonat | THF | 29,1 | Schlauch zieht ab |
| | 2,98 x 4,18 | EVA | Copolyester | THF | 23,4 | Schlauch zieht ab |
| | 2,98 x 4,18 | EVA | Copolyester | 50T MEK / 50T CH | 15,7 | Schlauch zieht ab |

**Tabelle 2: Messung der Biegesteifigkeit verschiedener einschichtiger und zweischichtiger Schläuche**

| | **Material** | **Biegesteifigkeit (mN)** |
|---|---|---|
| Versuchsreihe 1 | PVC | 0,120 |
| Versuchsreihe 2 | LDPE/S-TPE | 0,713 |
| Versuchsreihe 3 | EVA/Copolyesterether | 0,308 |
| Versuchsreihe 4 | EVA/S-TPE | 0,246 |
| Versuchsreihe 5 | EVA/Copolyester | 1,206 |

### Bezugszeichenliste

- 10: Schlauch
- 10': Schlauch
- 10": Schlauch
- 12: äußere Schicht aus S-TPE
- 14: Schicht aus EVA
- 14': Schicht aus EVA
- 14": Schicht aus EVA
- 16: innere Schicht aus S-TPE
- 20: äußere Oberfläche (der äußeren Schicht 12)
- 22: äußere Oberfläche (der Schicht 14, 14', 14")
- 24: innere Oberfläche (der Schicht 14, 14', 14")
- 26: innere Oberfläche (der inneren Schicht 16)
- Dᵢ: Innendurchmesser
- Dₐ: Außendurchmesser
- W₁₂: Dicke der Schicht 12
- W₁₄: Dicke der Schicht 14
- W_{14'}: Dicke der Schicht 14'
- W_{14"}: Dicke der Schicht 14"
- W₁₆: Dicke der Schicht 16

## Patentansprüche

1. Ein Schlauch (10; 10'; 10") zum Anschluss an einen medizinischen Behälter mit einer Schlauchwand, die aus mindestens zwei Schichten (12, 14; 14, 16) besteht, **dadurch gekennzeichnet, dass** mindestens eine Schicht (12; 16) ein Styrol-haltiges thermoplastisches Polymer, Copolyester, Copolyesterether oder Cyclo-Olefin-Copolymer enthält und mindestens eine Schicht (14, 14', 14") Ethylen-Vinylacetat-Copolymer (EVA), ein transparentes Thermoplastisches Polybuten und/oder Polystyrol-Polyethylenbutylen-Polystyrol (SEBS) enthält, wobei das Styrol-haltige thermoplastische Polymer ausgewählt ist aus Styrol-Butadien-Block-Copolymer (SBC), Polystyrol-Polybutadien-Polystyrol (SBS), Polystyrol-Polyisopren-Polystyrol (SEPS), Polystyrol-Isopren/Butadien-Polystyrol (SEEPS) und Polymethylmethacrylat-Polybutadien-Polystyrol (MBS) Blockcopolymer.

2. Schlauch nach Anspruch 1, **dadurch gekennzeichnet, dass** der Vinylacetat (VA)-Gewichtsanteil des Ethylen-Vinylacetat-Copolymers (EVA) 10% bis 30%, vorzugsweise 14% bis 28% ist.

3. Schlauch (10) nach Anspruch 1, **dadurch gekennzeichnet, dass** das Thermoplastische Polybuten oder SEBS der mindestens einen Schicht (14, 14', 14") bei der Herstellung als Additiv mit einem Gewichtsanteil von 1% bis 50%, vorzugsweise 10% bis 20%, hinzugefügt ist.

4. Schlauch (10) nach einem der Ansprüche 1 bis 3, **dadurch gekennzeichnet, dass** die äußere (12) der mindestens zwei Schichten (12, 14) das Styrol-haltige thermoplastische Polymer, Copolyester, Copolyesterether oder Cyclo-Olefin-Copolymer enthält.

5. Schlauch (10') nach einem der Ansprüche 1 bis 3, **dadurch gekennzeichnet, dass** die innere (16) der mindestens zwei Schichten (14, 16) das Styrol-haltige thermoplastische Polymer, Copolyester, Copolyesterether oder Cyclo-Olefin-Copolymer enthält.

6. Schlauch nach einem der vorhergehenden Ansprüche, **dadurch gekennzeichnet, dass** die das Polymer ausgewählt aus Styrol-haltigem thermoplastischen Polymer, Copolyester, Copolyesterether oder Cyclo-Olefin-Copolymer enthaltende Schicht (12; 16) eine Dicke (W12; W16) aufweist, die geringer ist als die Dicke (W14; W14') der anderen Schicht (14; 14') der mindestens zwei Schichten.

7. Schlauch (10") nach einem der Ansprüche 1 bis 3, **dadurch gekennzeichnet, dass** die Schlauchwand aus mindestens drei Schichten (12, 14", 16) besteht, und dass die äußere (12) und die innere (16) der mindestens drei Schichten ein Styrol-haltiges thermoplastisches Polymer (S-TPE), Copolyester, Copolyesterether oder Cyclo-Olefin-Copolymer enthalten.

8. Schlauch nach Anspruch 7, **dadurch gekennzeichnet, dass** die äußere und die innere, das Styrol-haltige thermoplastische Polymer enthaltende Schicht (12, 16) jeweils eine Dicke (W12; W16) aufweisen, die geringer ist als die Dicke (W14") einer anderen Schicht (14") der mindestens drei Schichten.

9. Schlauch nach einem der vorhergehenden Ansprüche, **dadurch gekennzeichnet, dass** die das Styrol-haltige thermoplastische Polymer, Copolyester, Copolyesterether oder Cyclo-Olefin-Copolymer enthaltende Schicht (12; 16) eine Dicke (W12; W16) von 0,01 bis 0,30 mm aufweist.

10. Schlauch nach einem der vorhergehenden Ansprüche, **dadurch gekennzeichnet, dass** der Schlauch einen Innendurchmesser (Dᵢ) von 1,0 bis 6,0 mm mit einer Toleranz von ± 0,1 mm aufweist.

11. Schlauch nach einem der vorhergehenden Ansprüche, **dadurch gekennzeichnet, dass** der Schlauch einen Außendurchmesser (Dₐ) von 2,0 bis 8,0 mm mit einer Toleranz von + 0,1 mm und - 0,3 mm aufweist.

12. Schlauch nach einem der vorhergehenden Ansprüche, **dadurch gekennzeichnet, dass** der Schlauch eine Gesamt-Wanddicke von 0,3 mm bis 1,0 mm mit einer Toleranz von ± 0,05 mm aufweist.

13. Medizinischer Behälter, insbesondere für die enterale oder die parenterale Ernährung, **gekennzeichnet durch** mindestens einen Ansatz aus einem Schlauch (10, 10', 10") nach einem der vorhergehenden Ansprüche.

14. Medizinischer Behälter nach Anspruch 13, **dadurch gekennzeichnet, dass** das Material, aus dem der Behälter hergestellt ist, Ethylen-Vinylacetat-Copolymer (EVA) enthält und dass der Behälter einen als Ansatzstutzen ausgebildeten Konnektor aufweist, wobei in den Ansatzstutzen ein Schlauch (10, 10") nach Anspruch 1 oder 5 eingesteckt ist, und wobei insbesondere die Innenseite des Ansatzstutzens mit einer äußeren Schicht (12) des Schlauchs (10, 10") verschweißt oder verklebt ist.

15. Medizinischer Behälter nach Anspruch 13, **dadurch gekennzeichnet, dass** das Material, aus dem der Behälter hergestellt ist, Ethylen-Vinylacetat-Copolymer (EVA) enthält und dass der Behälter als Ansatz einen dornförmig ausgebildeten Konnektor umfasst, auf den ein Schlauch (10', 10") nach einem der vorhergehenden Ansprüche, insbesondere nach Anspruch 3 oder 5 aufgeschoben ist, wobei insbesondere die Außenseite des dornförmig ausgebildeten Konnektors mit einer inneren Schicht (16) des Schlauchs (10', 10") verschweißt oder verklebt ist.

## Claims

1. A tube (10; 10'; 10") for connection to a medical container with a tube wall, which consists of at least two layers (12, 14; 14, 16), **characterized in that** at least one layer (12; 16) contains a styrene-containing thermoplastic polymer, copolyester, copolyester ether or cyclic olefin copolymer and at least one layer (14, 14', 14") contains ethylene-vinyl acetate copolymer (EVA), a transparent thermoplastic polybutene and/or polystyrene-polyethylene-butylene-polystyrene (SEBS), wherein the styrene-containing thermoplastic polymer is selected from styrene-butadiene block copolymer (SBC), polystyrene-polybutadiene-polystyrene (SBS), polystyrene-polyisoprene-polystyrene (SEPS), polystyrene-isoprene/butadiene-polystyrene (SEEPS) and polymethyl-methacrylate-polybutadiene-polystyrene (MBS) block copolymer.

2. The tube according to claim 1, **characterized in that** the vinyl acetate (VA) weight portion of the ethylene-vinyl acetate copolymer (EVA) is 10% to 30%, preferably 14% to 28%.

3. The tube (10) according to claim 1, **characterized in that** the thermoplastic polybutene or SEBS is added to the at least one layer (14, 14', 14") during production as an additive with a weight portion of 1% to 50%, preferably 10% to 20%.

4. The tube (10) according to any one of claims 1 to 3, **characterized in that** the outer (12) of the at least two layers (12, 14) contains the styrene-containing thermoplastic polymer, copolyester, copolyester ether or cyclic olefin copolymer.

5. The tube (10') according to any one of claims 1 to 3, **characterized in that** the inner (16) of the at least two tubes (14, 16) contains the styrene-containing thermoplastic polymer, copolyester, copolyester ether or cyclic olefin copolymer.

6. The tube according to any one of the preceding claims, **characterized in that** the layer (12; 16) containing the polymer selected from styrene-containing thermoplastic polymer, copolyester, copolyester ether or cyclic olefin copolymer has a thickness (W12; W16) that is smaller than the thickness (W14; W14') of the other layer (14; 14') of the at least two layers.

7. The tube (10") according to any one of claims 1 to 3, **characterized in that** the tube wall consists of at least three layers (12, 14", 16), and that the outer (12) and the inner (16) of the at least three layers contain a styrene-containing thermoplastic polymer (S-TPE), copolyester, copolyester ether or cyclic olefin copolymer.

8. The tube according to claim 7, **characterized in that** the outer and the inner layer (12, 16) containing the styrene-containing thermoplastic polymer each have a thickness (W12; W16) that is smaller than the thickness (W14") of another layer (14") of the at least three layers.

9. The tube according to any one of the preceding claims, **characterized in that** the layer (12; 16) containing the styrene-containing thermoplastic polymer, copolyester, copolyester ether or cyclic olefin copolymer has a thickness (W12; W16) of 0.01 to 0.30 mm.

10. The tube according to any one of the preceding claims, **characterized in that** the tube has an inner diameter (Dᵢ) of 1.0 to 6.0 mm with a tolerance of ±0.1 mm.

11. The tube according to any one of the preceding claims, **characterized in that** the tube has an outer diameter (Dₐ) of 2.0 to 8.0 mm with a tolerance of +0.1 mm and -0.3 mm.

12. The tube according to any one of the preceding claims, **characterized in that** the tube has a total wall thickness of 0.3 to 1.0 mm with a tolerance of ±0.05 mm.

13. A medical container, in particular for enteral or parenteral nutrition, **characterized by** at least one attachment of a tube (10, 10', 10") according to any one of the preceding claims.

14. The medical container according to claim 13, **characterized in that** the material from which the container is manufactured contains ethylene-vinyl acetate copolymer (EVA) and that the container has a connector formed as an adaptor piece, wherein a tube (10, 10") according to Claim 1 or 5 is inserted into the adaptor piece, and wherein the inside of the adaptor piece in particular is welded or bonded to an outer layer (12) of the tube (10, 10").

15. The medical container according to Claim 13, **characterized in that** the material from which the container is manufactured contains ethylene-vinyl acetate copolymer (EVA) and that the container comprises as an attachment a cone-shaped connector, onto which a tube (10, 10") according to any one of the preceding claims, in particular according to Claim 3 or 5, is pushed, wherein the outside in particular of the cone-shaped connector is welded or bonded to an inner layer (16) of the tube (10', 10").

## Revendications

1. Tuyau (10 ; 10' ; 10") destiné à être raccordé à un récipient médical, comprenant une paroi de tuyau constituée d'au moins deux couches (12, 14 ; 14, 16), **caractérisé en ce qu'**au moins une couche (12 ; 16) contient un polymère thermoplastique contenant du styrène, un copolyester, un éther de copolyester ou un copolymère cyclo-oléfinique, et au moins une couche (14, 14', 14") contient un copolymère éthylène-acétate de vinyle (EVA), un polybutène thermoplastique transparent et/ou du polystyrène-polyéthylène-butylène-polystyrène (SEBS), le polymère thermoplastique contenant du styrène étant choisi parmi les copolymères à blocs styrène-butadiène (SBC), polystyrène-polybutadiène-polystyrène (SBS), polystyrène-polyisoprène-polystyrène (SEPS), polystyrène-isoprène/butadiène-polystyrène (SEEPS) et les copolymères à blocs polyméthylméthacrylate-polybutadiène-polystyrène (MBS).

2. Tuyau selon la revendication 1, **caractérisé en ce que** la proportion en poids d'acétate de vinyle (VA) du copolymère éthylène-acétate de vinyle (EVA) est de 10 % à 30 %, de préférence de 14 % à 28 %.

3. Tuyau (10) selon la revendication 1, **caractérisé en ce que** le polybutène thermoplastique ou le SEBS est ajouté comme additif à ladite au moins une couche (14, 14', 14") pendant la fabrication dans une proportion en poids de 1 % à 50 %, de préférence de 100 % à 20 %.

4. Tuyau (10) selon l'une des revendications 1 à 3, **caractérisé en ce que** la couche extérieure (12) desdites au moins deux couches (12, 14) contient le polymère thermoplastique contenant du styrène, le copolyester, l'éther de copolyester ou le copolymère cyclo-oléfinique.

5. Tuyau (10') selon l'une des revendications 1 à 3, **caractérisé en ce que** la couche intérieure (16) desdites au moins deux couches (14, 16) contient le polymère thermoplastique contenant du styrène, le copolyester, l'éther de copolyester ou le copolymère cyclo-oléfinique.

6. Tuyau selon l'une des revendications précédentes, **caractérisé en ce que** la couche (12 ; 16) contenant le polymère choisi parmi un polymère thermoplastique contenant du styrène, un copolyester, un éther de copolyester ou un copolymère cyclo-oléfinique présente une épaisseur (W12 ; W16) qui est inférieure à l'épaisseur (W14 ; W14') de l'autre couche (14 ; 14') desdites au moins deux couches.

7. Tuyau (10") selon l'une des revendications 1 à 3, **caractérisé en ce que** la paroi de tuyau est constituée d'au moins trois couches (12, 14", 16) et **en ce que** la couche extérieure (12) et la couche intérieure (16) desdites au moins trois couches contiennent un polymère thermoplastique contenant du styrène (S-TPE), un copolyester, un éther de copolyester ou un copolymère cyclo-oléfinique.

8. Tuyau selon la revendication 7, **caractérisé en ce que** les couches extérieure et intérieure (12, 16) contenant le polymère thermoplastique contenant du styrène présentent chacune une épaisseur (W12 ; W16) qui est inférieure à l'épaisseur (W14") d'une autre couche (14") desdites au moins trois couches.

9. Tuyau selon l'une des revendications précédentes, **caractérisé en ce que** la couche (12 ; 16) contenant le polymère thermoplastique contenant du styrène, le copolyester, l'éther de copolyester ou le copolymère cyclo-oléfinique présente une épaisseur (W12 ; W16) de 0,01 à 0,30 mm.

10. Tuyau selon l'une des revendications précédentes, **caractérisé en ce que** le tuyau présente un diamètre intérieur (Dᵢ) de 1,0 à 6,0 mm avec une tolérance de ± 0,1 mm.

11. Tuyau selon l'une des revendications précédentes, **caractérisé en ce que** le tuyau présente un diamètre extérieur (Dₐ) de 2,0 à 8,0 mm avec une tolérance de + 0,1 mm et - 0,3 mm.

12. Tuyau selon l'une des revendications précédentes, **caractérisé en ce que** le tuyau présente une épaisseur de paroi totale de 0,3 mm à 1,0 mm avec une tolérance de ± 0,05 mm.

13. Récipient médical, en particulier pour la nutrition entérale ou parentérale, **caractérisé par** au moins un embout formé d'un tube (10, 10', 10") selon l'une des revendications précédentes.

14. Récipient médical selon la revendication 13, **caractérisé en ce que** le matériau à partir duquel le récipient est fabriqué contient un copolymère éthylène-acétate de vinyle (EVA), et **en ce que** le récipient présente un connecteur réalisé sous la forme d'un raccord à embout, dans lequel un tuyau (10, 10") selon la revendication 1 ou 5 est inséré dans le raccord à embout, et dans lequel notamment le côté intérieur du raccord à embout est soudé ou collé à une couche extérieure (12) du tuyau (10, 10").

15. Récipient médical selon la revendication 13, **caractérisé en ce que** le matériau à partir duquel le récipient est fabriqué contient un copolymère éthylène-acétate de vinyle (EVA), et **en ce que** le récipient comprend, en tant qu'embout, un connecteur réalisé en forme d'épine sur lequel est enfilé un tuyau (10', 10") selon l'une des revendications précédentes, en particulier selon la revendication 3 ou 5, dans lequel notamment le côté extérieur du connecteur réalisé en forme d'épine est soudé ou collé à une couche intérieure (16) du tuyau (10', 10").
